# EUROPEAN PATENT APPLICATION

(11) **EP 2 578 220 A1**
(43) Date of publication of application: **10.04.2013**
(21) Application number: 11786351.4
(22) Date of filing: 27.05.2011
(51) Int. Cl.: A61K 31/732, A61K 31/711, A61P 31/16

(54) **INFLUENZA VIRUS INFECTION INHIBITOR**

(30) Priority: 28.05.2010 JP 2010122582
(71) Applicant: Y's Corporation, Tokyo 171-0032 (JP)
(72) Inventor: KOIDE, Masafumi, Nagoya-shi Aichi 468-0051 (JP)
(74) Representative: Delorme, Nicolas
(86) International application number: PCT/JP2011/002973
(87) International publication number: WO 2011/148648

(57) **Abstract**

An effective inhibitor of influenza virus infection on cells can be achieved by safety components for humans. The inhibitor of influenza virus infection consists of pectin and polynucleotide as main components and has an effect that inhibits adhesion between hemagglutinin and sialic acid.

## Description

### TECHNICAL FIELD

The present invention relates to inhibitors of influenza virus infection.

### BACKGROUND ART

Influenza is caused by infection with influenza viruses which are RNA viruses belonging to the orthomyxovirus family. An influenza pandemic often occurred, and caused many deaths. Therefore, various studies of therapy and prevention of influenza have been done. Oseltamivir etc. have been known as a therapeutic agent for influenza. These therapeutic agents inhibit neuraminidase that influenza viruses have, and make the influenza viruses hard to be released from the infected cells. While oseltamivir is used for prevention of influenza, it does not have a function of directly inhibiting infection of cells with influenza viruse s .

The most direct method for preventing influenza virus infection in the body is to physically prevent by masks etc. However, it is difficult to completely prevent infections from invisible and fine viruses to the body.

Administration of a vaccine is said to be effective for prevention of onset and symptomatic relief of influenza. However, an influenza virus frequently mutates, and there are many subtypes thereof. Therefore, an outstanding advantage cannot be expected as long as the type of an administered vaccine is matched with the type of epidemic influenza virus. Even if an antibody against the influenza virus is formed in the blood by the vaccine, it does not inhibit the influenza virus infection on mucosal epithelial cell in the airway. Inoculation of a vaccine may cause side effects, such as an allergy etc.

Various studies have been done to find substances which are hard to be affected by mutation of influenza viruses and which directly inhibit infection of cells with influenza viruses. In terms of safe in humans, components derived from various natural products have been considered. Anti-influenza drugs etc. using various plant extracts, such as tea, orange, cacao, etc., have been developed (for example, see Patent Documents 1-3).

### CITATION LIST

### PATENT DOCUMENT

PATENT DOCUMENT 1: Japanese Patent Publication No. 2004-59463
PATENT DOCUMENT 2: Japanese Patent Publication No. 2005-343836
PATENT DOCUMENT 3: Japanese Patent Publication No. 2007-223970

### SUMMARY OF THE INVENTION

### TECHNICAL PROBLEM

In order to defeat various types of influenza viruses, it has been desired to develop materials having an effect of universally inhibiting infection with influenza viruses. Most effective components derived from plant extracts are not clearly specified. Therefore, due to variations of habitats of the plants, variations of extraction processes, etc., the inhibiting effect on influenza virus infection may vary. All materials derived from plants are not necessarily safe in humans. Therefore, it is preferable to specify materials in terms of safety.

The present inventor has found that components which have been confirmed to be safe in humans can inhibit infection of cells with influenza viruses. In view of the finding, it is an object of the present invention to achieve a safer inhibitor which can efficiently inhibit influenza virus infection.

### SOLUTION TO THE PROBLEM

Specifically, an inhibitor of influenza virus infection according to the present invention is a composition containing pectin and polynucleotide which is a macromolecular chain of nucleic acid as main components and the inhibitor has activity of blocking adhesion between hemagglutinin of influenza virus and sialic acid on cells.

The present inventor has found that pectin which is a polysaccharide and a polynucleotide which is a strongly, negatively charged macromolecule can efficiently inhibits infection of cells with influenza viruses. The pectin and the polynucleotide are food components, and are safe in humans. They are materials which can be stably obtained. They also have inhibiting activity of blocking adhesion between hemagglutinin and sialic acid on cells, and unlike an inhibitor of inhibiting neuraminidase, they can prevent influenza viruses from entering host cells, and therefore, they can directly inhibit influenza virus infection on mucosal epithelial cells etc. in the airway.

In the inhibitor of influenza virus infection of the present invention, a concentration of the composition of the pectin and the polynucleotide may be 0.04% or more, a concentration of the pectin may be 0.01 % or more, and a concentration of the nucleic acid may be 0.01% or more.

In the inhibitor of influenza virus infection of the present invention, the pectin may have a molecular weight of 50000 Da or more, and the polynucleotide may have a molecular weight of 10000 Da or more.

In the inhibitor of influenza virus infection of the present invention, the polynucleotide may be a deoxyribonucleic acid.

### ADVANTAGES OF THE INVENTION

According to the inhibitor of influenza virus infection of the present invention, infection of cells with influenza viruses can be efficiently inhibited.

### BRIEF DESCRIPTION OF THE DRAWINGS

FIG. 1 is a photograph showing results of a hemagglutination inhibition test using an inhibitor of infection according to an embodiment.
FIG. 2 is a chart showing the results of the hemagglutination inhibition test using the inhibitor of infection according to the embodiment.
FIG. 3 is a graph showing a relationship between a concentration of the inhibitor of infection according to the embodiment and an infection inhibition rate.

### DESCRIPTION OF EMBODIMENTS

An inhibitor of influenza virus infection according to an embodiment includes pectin and nucleic acid. The pectin is a polysaccharide constituting cell walls of plants, and is included in almost all plants. Therefore, pectin has a different structure and molecular weight depending on the plant. However, pectin basically includes, as main components, homogalacturonan which is a homopolymer of galacturonic acid, and rhamnogalacturonan which is formed by bonding rhamnose and galacturonic acid to each other. A monosaccharide or a polysaccharide is bonded to rhamnose residues of rhamnogalacturonan as a side chain. A carbohydrate constituting the side chain is arabinose, xylose, fucose, and rhamnose, etc. At least part of the galacturonic acid is esterified.

Materials made of apples, citrus fruits, sugar beets, etc., have been known as commercially available pectins, and the molecular weights of them are in the range of approximately 50000 Da - 360000 Da. While any types of pectins can be utilized for the inhibitor of influenza virus infection in the embodiment, the molecular weight thereof is preferably 50000 Da or more, and is more preferably 100000 Da or more since a larger molecular weight allows more influenza viruses to be trapped.

The nucleic acid is a macromolecule (polynucleotide) obtained by bonding a plurality of nucleotides, each composed of a pentose, a phosphoric acid, and a base, to each other. The nucleic acid including deoxyribose as the pentose is a deoxyribonucleic acid (DNA), and the nucleic acid including ribose as the pentose is a ribonucleic acid (RNA). The base generally included in the DNA is adenine, guanine, cytosine, or thymine. The base generally included in the RNA is adenine, guanine, cytosine, or uracil. Each of the nucleotides in the nucleic acid includes at least a negative charge.

The inhibitor, including pectin and nucleic acid, of infection is used, thereby making it possible to efficiently inhibit influenza virus infection on cells. It is not clear why the inhibitor obtained by mixing the pectin and the nucleic acid efficiently inhibits influenza virus infection on cells. It has been known that influenza viruses enter cells through hemagglutinin existing on the surface of a virus (hereinafter referred to as hemagglutinin activity). The pectin which has a hydrophilic side chain homologous to a sialic acid, and a hydrophobic ester group having an affinity for virus envelopes cooperatively acts with the nucleic acid which is a strongly, negatively charged macromolecule, and the cooperative action may inhibit hemagglutinin activity, and the access of influenza viruses to the mucosal surface. It is found that the inhibitor of infection in the embodiment can inhibit the hemagglutination reaction by influenza viruses, and based on the finding, it is also strongly assumed that the inhibitor of infection in the embodiment inhibits hemagglutinin activity. In this way, the inhibitor of infection in the embodiment inhibits infection of influenza viruses on cells. Inhibition mechanism of infection with influenza viruses is different from a neuraminidase inhibitor inhibiting influenza viruses, which have grown in the inside of cells, from being released to the outside of the cells.

The nucleic acid may be a macromolecule having a negative charge, and the sequence of the bases are not limited. The effect of nucleic acid is not affected by the saccharide, and may be a DNA or an RNA. A DNA is preferable in terms of stability. The nucleic acid does not have to be a polymer composed of only nucleotides, and there is no problem even if the nucleic acid is mixed with or bonded to other molecules such as protein etc. While the molecular weight of the nucleic acid does not have to be fixed, it is preferably 10000 Da or more, and is more preferably 100000 Da or more since a larger molecular weight allows more influenza viruses to be captured. While it is preferable that the nucleic acid have a higher molecular weight, the molecular weights of a commercially available nucleic acid is generally 1000000 Da or less, and at most approximately 3000000 Da. The DNA having such a molecular weight can be utilized without problems.

The concentration of the inhibitor, which is a composition of pectin and nucleic acid, of infection is preferably about 0.04% by mass or more, and is more preferably about 0.09% by mass or more. The higher concentration of the composition of the pectin and the nucleic acid allows more influenza viruses to be captured. However, the concentration of the composition of the pectin and the nucleic acid is too large, the viscosity thereof is increased, and therefore, the concentration is preferably about 1% by mass or less, and is more preferably about 0.5% by mass or less. Since the pectin and the nucleic acid cooperatively act with each other, it is preferable that one of concentrations of the pectin or the nucleic acid should not be extremely lower, and the mass ratio between the pectin and the nucleic acid in the inhibitor of infection should be in the range of approximately 1:5-5 :1. There is no problem even if the large amount of the pectin or the large amount of the nucleic acid is included. It is preferable that each of the concentrations of the pectin and the nucleic acid should not be under 0.01 % by mass.

The inhibitor, including the pectin and the nucleic acid, of influenza virus infection is supplied to mucosal cells in the nose or the oral cavity, thereby making it possible to efficiently inhibit influenza virus infection (invasion) on the mucosal cells. Since the pectin and the nucleic acid are food components which are safe in humans, there is no problem if they are used to the human body. Therefore, in the embodiment, methods for supplying the inhibitor of infection to mucosal cells in the nose or the oral cavity are not limited. For example, the inhibitor may be sprayed into the nostrils and the oral cavity by a spray etc., and may be introduced into the nostrils and the oral cavity by using an inhaler etc. The inhibitor may be introduced into the oral cavity as a mouthwash to be supplied to mucosal cells. These cases may utilize a solution or a suspension including the pectin and the nucleic acid in water as solvent, and a stabilizer, a dispersant, an emulsifier, and a diluent, etc., may be added into the solvent as appropriate. The inhibitor of infection in the embodiment may be added into troches or chewing gum, etc., to be introduced into the oral cavity, thereby being supplied to the mucosal cells. When it is introduced into the oral cavity or the nostril, a flavor may be added.

The inhibitor of infection may be orally administered to inhibit the inside of the bowel from being infected with influenza viruses. In this case, capsules, tablets, granules, or syrup, etc., allowing the inhibitor of infection to be delivered to the inside of the bowel may be utilized.

If the inhibitor is supplied as inhalants, mouthwashes, troches or other drugs, to mucosal cells or cells in the bowel, the concentration of the inhibitor of infection in the supplied portion may be in the range of about 0.04% by mass - about 1% by mass, and may be preferably in the range of about 0.09% by mass - about 0.5% by mass.

The inhibitor of infection may be allowed to act on influenza viruses existing in open atmosphere. In this case, the inhibitor of infection may be released to the air by spraying etc. The inhibitor of infection may be locally sprayed onto a floor or a doorknob touched by the hand, etc.

### (Example)

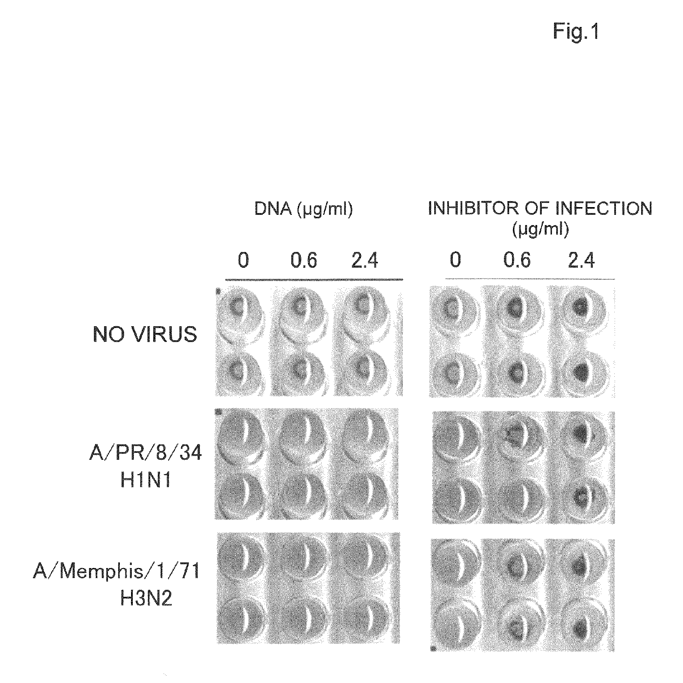

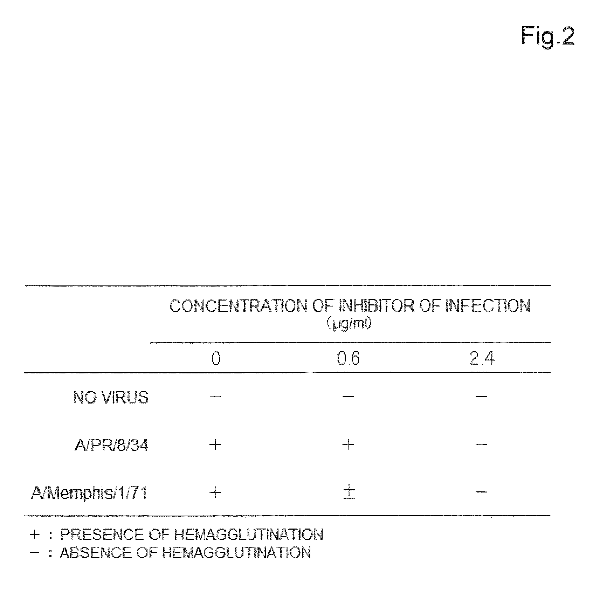

One example of the inhibitor, including the pectin and the nucleic acid, of influenza virus infection will be shown below. FIG. 1 and FIG. 2 show results of a hemagglutination inhibition test. In FIG. 1 and FIG. 2, pectin (produced by CP Kelo: GENU pectin (citrus) type USP-L, a galacturonic acid ratio of 74% or more, a methoxyl group ratio of 6.7 or more) derived from skins of citrus fruits was used as the pectin. An nucleic acid (produced by Daiwa Fine Chemicals Co., Ltd.: DNA-Na, Lot: DN-JD0603) derived from salmon was used as the nucleic acid. The mass ratio between the pectin and the DNA in the inhibitor of infection was 1:1.

The hemagglutination inhibition test was performed as shown below by following a general method. Isolated red blood cell suspensions from a guinea pig, influenza viruses (A/PR/8/34 (H1N1 type) strain or A/Memphis/1/71 (H3N2 type) strain), and inhibitors of infection having a predetermined concentration in phosphate buffered saline (PBS) were added on wells of a microtiter plate. After incubation for a predetermined period of time, the evaluation of hemagglutination was done by visual and microscopic observation. The influenza virus-free wells were considered as a negative control. As comparative examples, only an DNA was added instead of the inhibitor of infection, and a similar test was performed.

As shown in FIG. 1, hemagglutination does not occur in the negative control on which the influenza viruses were not added, and only sedimentation of red blood cells were confirmed. In the wells on which the inhibitor of infection was not added and the wells on which only the DNA was added, strong hemagglutination was observed. In contrast, in the wells on which the inhibitor of infection was added, hemagglutination was clearly inhibited. A slightly higher hemagglutination inhibition effect was shown on the A/Memphis/1/71 strain which was the H3N2 type, and a similar hemagglutination inhibition effect was also shown on the A/PR/8/34 strain which was the H1N1 type. This shows that, unlike antibodies, the inhibitor of infection in the embodiment has an effect of inhibiting hemagglutinin activity regardless of the types of influenza viruses.

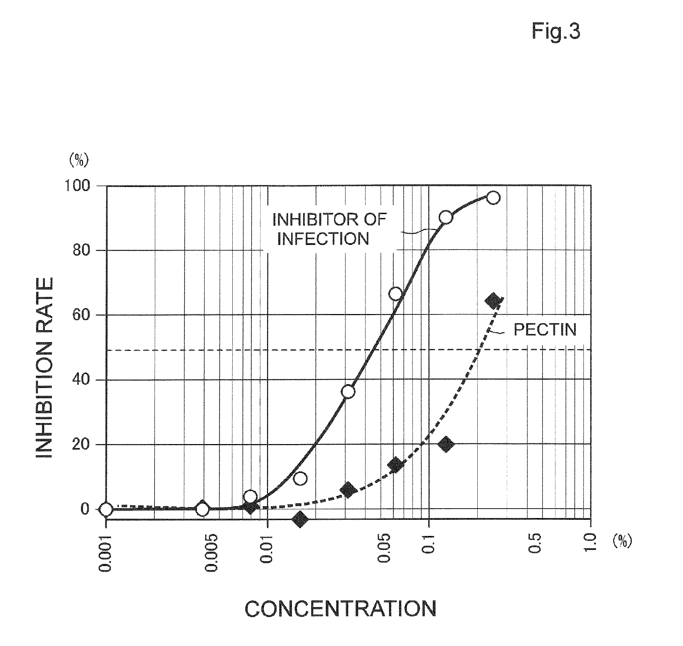

FIG. 3 shows the inhibition effect of influenza virus infection by the inhibitor of infection including the pectin and the nucleic acid. The infection inhibition effect was evaluated as indicated below. After Madin-Darby canine kidney cells (MDCK cells) was plated on a serum-free culture medium, the inhibitor of infection was added so as to be at a predetermined concentration. After the MDCK cells were cultured for one hour at a temperature of 37°C, the culture medium was replaced with a culture medium including influenza viruses, and was cultured for twenty hours at a temperature of 34°C. The A/Memphis/1/71 strain was used as the influenza virus after the strain was left for one hour at a temperature of 4°C in a serum-free culture medium in which the inhibitor of infection was added at a predetermined concentration. After the cultivation, the serum-free culture medium was removed, and the MDCK cells were fixed using methanol, and then, the cells infected with the influenza viruses were immunostained using an antiviral antibody to measure the amount of the staining. By assuming the amount of the staining in the case where the inhibitor of infection were not added to be an infection rate of 100%, the infection inhibition rate (inhibition rate against infection) was obtained from the amount of staining. Similar procedures were performed in the case where only the pectin was added without including the nucleic acid as a comparative example. The same inhibitor of infection as that used in the hemagglutination inhibition test was used.

As shown in FIG. 2, in the case where only the pectin was added without including the nucleic acid, the infection inhibition rate (inhibition rate against infection) was about 50% when the concentration of the pectin was about 0.2% by mass. In contrast, in the case where the inhibitor of infection included the pectin and the nucleic acid, the infection inhibition rate (inhibition rate against infection) was about 50% when the concentration of the inhibitor was about 0.04% by mass, and the infection inhibition rate (inhibition rate against infection) was about 80% when the concentration of the inhibitor was about 0.09% by mass. The infection inhibition rate (inhibition rate against infection) was about 96% when the concentration of the inhibitor was about 0.25% by mass, which is far higher than that in the case of using only the pectin.

### INDUSTRIAL APPLICABILITY

The inhibitor of infection according to the present invention can efficiently inhibit of influenza virus infection on cells, and is useful as an inhibitor of influenza virus infection etc.

## Claims

1. A composition for inhibiting adhesion between hemagglutininof influenza virus and sialic acid, the composition mainly containing pectin and polynucleotide.

2. The inhibitor of claim 1, wherein
a concentration of the composition of the pectin and the polynucleotide is 0.04% or more,
a concentration of the pectin is 0.01 % or more, and
a concentration of the polynucleotide is 0.01 % or more.

3. The inhibitor of claim 1 or 2, wherein
the pectin has a molecular weight of 50000 Da or more, and
the polynucleotide has a molecular weight of 10000 Da or more.

4. The inhibitor of any one of claims 1-3, wherein
the polynucleotide is a deoxyribonucleic acid.
